# EUROPEAN PATENT APPLICATION

(11) **EP 4 718 070 A1**
(43) Date of publication of application: **01.04.2026**
(21) Application number: 24306587.7
(22) Date of filing: 27.09.2024
(51) Int. Cl.: G01N 33/58, C08L 83/04, A61K 47/34, G01N 33/68

(54) **METHOD OF EVALUATING THE INTERACTIONS BETWEEN A LAYER OF SILICONE OIL DEPOSITED ON A SUBSTRATE AND A COMPOUND OF INTEREST**

(71) Applicant: Becton Dickinson France, 38800 Le Pont-de-Claix (FR); Institut Polytechnique de Grenoble, 38000 Grenoble (FR); Université Grenoble Alpes, 38400 Saint-Martin-d'Hères (FR); Centre National de la Recherche Scientifique, 75016 Paris (FR)
(72) Inventor: RODRIGUEZ, Mathilde, 38500 COUBLEVIE (FR); BRUNET, Claire, 38000 GRENOBLE (FR); BRUCKERT, Franz, 38100 Grenoble (FR); WEIDENHAUPT, Marianne, 38134 St Joseph de Rivière (FR)
(74) Representative: Germain Maureau

(57) **Abstract**

The present invention relates to a method of evaluating the interactions between a layer of silicone oil deposited on a substrate and a compound of interest which is contained in a solution, said solution being in contact with the silicone oil, which comprises the following steps:
1) preparing a mixture containing the silicone oil and an evaporable solvent,
2) contacting the mixture with the substrate,
3) evaporating the solvent to obtain a substrate coated with a layer of the silicone oil,
4) contacting the layer of the silicone oil with the solution,
5) evaluating at least one of the following parameters i), ii) and iii) :
i) the adsorption of the compounf of interest on the layer of the silicone oil,
ii) the degradation of the compound of the interest,
iii) the degradation of the layer of the silicone oil.

## Description

The present invention relates to a method for evaluating the interactions between a layer of silicone oil deposited on a substrate and at least one compound of interest (for example a drug) present in a solution which is contact with said layer of silicone oil.

Pharmaceutical solutions containing monoclonal antibodies which are proteins are widely used for the immunotherapy treatment of several cancers (for example breast cancer or stomach cancer).

Pharmaceutical companies are increasingly using glass pre-filled syringes as an alternative to vial packaging for the delivery of such pharmaceutical solutions containing drug. The major purposes of using glass pre-filled syringes packaging are, among others, to:
- simplify the administration of the pharmaceutical solution by the caregiver,
- reduce the risk of microbial contamination,
- provide reduced injection cost with a lower dead volume, and
- preserve the compounds of interest (i.e. the drug, for example the monoclonal antibodies or vaccines) during the storage of the pharmaceutical solution until delivered safely for injection.

To ensure efficient gliding of the stopper during drug injection, ensure the injectability of the pharmaceutical solution and to attain optimal glass pre-filled syringes functionality, commonly polydimethylsiloxane (hereafter "PDMS") medical grade silicone oil is used as a lubricant to coat both barrel and stopper surfaces of the syringe.

However, this hydrophobic layer is a preferential adsorption site for drugs (such as monoclonal antibodies) and surfactants which are present in pharmaceutical solutions. Thus, it may affect the biotherapeutic effect of the pharmaceutical solution and lead to a release of silicone oil into the pharmaceutical solution and entail injectability issues.

More precisely, the implication of the silicone oil-water and air-water interfaces on drug aggregation and particle formation in pharmaceutical solutions is well-known. Drug exposure to such interfaces can lead to drugs adsorption, structural perturbation and aggregation. To minimize the effects of the silicone oil-liquid interface on drugs aggregation during agitation, surfactants are often included in the formulation of pharmaceutical solutions.

Surfactants have been presented to be relevant molecules to protect drug against interaction with hydrophobic surfaces and are now commonly used in formulations of pharmaceutical solutions, such as pharmaceutical solutions of monoclonal antibodies, to reduce biologic adsorption and aggregation in siliconized glass pre-filled syringes. Moreover, surfactants are used to stabilize the drug in the pharmaceutical solution.

However, if surfactants are effective to prevent drugs from degradation at interfaces, these amphiphilic molecules can also favor the interaction between the drugs and the silicone oil. Indeed, in combination with other variables (such as pH and buffer/tonicity agents...), the presence of surfactants in the pharmaceutical solution can promote silicone oil layer degradation via a complex set of phenomena summarized as "de-lub", "wet" and "wash" processes and trigger changes on silicone coating lubricant properties. Drugs interaction with silicone oil coating may lead to subvisible particle formation and impaired glass pre-filled syringe performances.

From this perspective, the evaluation of the interactions between the drug and the layer of silicone oil is pivotal for developing a glass pre-filled syringe containing a pharmaceutical solution before its launching on the market. Indeed, as explained above, it is crucial that :
- the compound of intest (i.e. the drug) contained in the pharmaceutical solution is not degraded during the storage in the pre-filled syringe and the administration of the drug to the patient so that the amount of drug which is administered to the patient is the appropriate dose of drug for obtaining the desired therapeutic effect,
- the layer of silicone oil is not degraded, because such a deterioration reduces the gliding performances and could generate issues of administration of the pharmaceutical solution that may lead to the administration of a different dose of the drug than the intended dose for the desired therapeutic effect.

More precisely, these interactions are non-specific. Indeed, these interactions are triggered by forces emanating from a combination of different properties of both the silicone oil and the interacting compounds of interest from the formulation, such as: chemistry, topography, charge, hydrophobicity, etc.

Therefore, a complex combination of interaction forces (such as Van der Waals, electrostatic, hydrophobic, hydrogen bonding, etc) and physico-chemical parameters (such as temperature, pH, ionic strength etc) govern these non-specific interactions.

A consequence of this non-specificity is that these interactions are poorly predictable and not transferable between different formulation-container combinations.

In other words, the evaluation of the interactions needs to be carried out for each combination of a specific layer of silicone oil and a specific pharmaceutical solution containing compounds of interest.

Thus, it explains why before launching a glass pre-filled syringe on the market, it is important to address the interactions between the layer of silicone oil and the compounds of interest (i.e. the drugs) present in the pharmaceutical solution contained in the syringe in order to be sure of the absence of the degradation of said compounds of interest and of said layer of silicone oil that would compromise the administration of the pharmaceutical solution and the intended therapeutic effect.

The evaluation of said interactions is carried out during the development of combined container-formulation product by optimizing the features of the layer of silicone oil and/or the formulation of the pharmaceutical solution.

To that end, and in particular for the development of glass pre-filled syringes, it may be necessary to carry out a lot of experiments that are long (and they ideally should cover the expected shelf life of the pharmaceutical solution), expensive (in particular as the result of the use of high amounts of the drug which often is costly and the need of many replicate experiments) and difficult (in particular as a result of the lab conditions). Additionally, for the case of glass pre-filled syringes, the syringe cylindrical shape does not always allow all direct standard measuring methods and therefore needs time-consuming sample preparation.

Thus, in particular for the development of pre-filled syringes, there is a need to have a method of evaluating the interactions between the layer of silicone oil and the drug of the pharmaceutical solution contained in the syringe which is easy to be carried-out, reliable and cost-effective and allows a lot of replicate experiments.

This need of a method for evaluating these interactions may be more generally formulated. Indeed, this would apply not only to a glass pre-filled syringe, but also to any substrate on which a layer of silicone oil is deposited (for example plastics and rubber) and to other devices (such as pump pipe, glass well-plates).

The inventors of the present invention surprisingly found a method for evaluating these interactions which fully complies with these above detailed objectives.

The invention concerns a method of evaluating the interactions between a layer of silicone oil deposited on a substrate and at least one compound of interest, preferably a drug, which is contained in a solution, preferably a pharmaceutical solution, said solution being in contact with the silicone oil, which comprises at least the following steps:
1) preparing a mixture containing at least the silicone oil and an evaporable solvent which is miscible with the silicone oil and compatible with the substrate,
2) contacting the mixture with the substrate,
3) evaporating the solvent to obtain a substrate coated with a layer of the silicone oil,
4) contacting the layer of the silicone oil with the solution containing the at least one compound of interest,
5) evaluating at least one of the following parameters i), ii) and iii) :
   i) the adsorption of the at least one compound of interest on the layer of the silicone oil,
   ii) the degradation of the at least one compound of the interest,
   iii) the release of the silicone oil in the solution,
wherein if the parameter iii) of the release of the silicone oil in the solution is evaluated, the silicone oil is labelled.

The method of evaluating according to the invention provides the following advantages:
- the steps 1 to 3) for depositing a layer of silicone oil is a very effective and reliable method for obtaining a qualititative and easily replicated layer of silicone oil on a substrate;
- thanks to the steps 1) to 3), the thickness and the amount of the layer of silicone oil may be precisely controlled to obtain the targeted thickness;
- the method is reliable, unexpensive and is easily carried out;
- the method may involve low sample volumes;
- the method allows the easy creation of a lot of replicates if necessary;
- as described below, the method may involve well-known assays for evaluating the adsorption and/or the degradation of the compound of interest.

Preferably, the solution is an aqueous solution.

Preferably, the solution is an aqueous pharmaceutical solution.

The solution may comprise at least one organic solvent (for example dimethyl sulfoxide).

In some embodiments of the invention, the solution may be an aqueous solution (preferably an aqueous pharmaceutical solution) which further comprises at least one organic solvent.

Preferably, the compound of interest is a drug. The drug may be selected from the groupe consisting of therapeutic proteins (such as for example monoclonal antibodies), vaccines (such as conjugate vaccines, viral vectors), drug-conjugates (such ad antibody drug conjugates, polymeric drug conjugates), cell and gene therapy, oligonucleotides, drug carriers (such as lipid nanoparticles) and oncolytic virus.

The material of the substrate may be any material on which a layer of silicone oil may be deposited. The material may be chosen from the group consisting of glass, rubber, plastics and metals. Preferably, the material of the substrate is glass.

The substrate may be any device or any part of a device on which a layer of silicone oil may be deposited. The substrate may be chosen from the group consisting of syringes, pump pipes, well plates (in particular glass bottom well plates), glass or plastics or rubber slices/plates, plunger rubber stoppers and needles. Preferably, the substrate is a glass bottom well plate or a syringe.

The silicone oil may be any kind of silicone oil.

Preferably, the silicone oil comprises a medical grade silicone oil.

In preferred embodiments of the invention, the silicone oil comprises a PDMS silicone oil.

In most preferred embodiments of the invention, the silicone oil comprises a medical grade PDMS silicone oil.

The silicone oil may have a viscosity comprised between 50 mm²/s and 1000 000 mm²/s at 25°C. For example, the viscosity of the silicone oil is 1000 mm²/s at 25°C.

The silicone oil may have a density comprised between 0.800 g/cm³ and 1.100 g/cm³ at 25°C. For example, the density of the silicone oil is 0.9700 g/cm³ at 25°C.

The silicone oil may have a refractive index comprised between 1.390 and 1.410. For example, the refractive index of the silicone oil is 1.400.

In preferred embodiments of the invention, the silicone oil is the silicone oil marketed by the firm Dow under the tradename of Dow Corning^{®} DC360.

If in step 5) of the method of evaluating, the release of the silicone oil in the solution is evaluated, the silicone is labelled.

Preferably, the silicone oil is labelled with fluorescent compounds.

The silicone oil may be labelled with a labelling rate that may not exceed 10%. According to the present invention, '"the labelling rate" represents the % in mass of the silicone oil which has been labelled, said % in mass being based on the total mass of silicone oil for which the labelling has been performed.

Preferably, the labelling rate may be comprised between 0.001% and 10%, more preferably between 0.01% and 2%. This range of labelling rate is convenient for fluorescence experiments in order to determine in a reliable manner the amount of silicone oil present in a sample of a solution (preferably a pharmaceutical solution). Indeed, if the rate of labelling the silicone oil is higher, the fluorescent compounds may be too close together and there is the risk of a quenching phenonemon that will distort the determination of silicone oil present in the solution.

Most preferably, the silicone oil is labelled with a fluorescent compound by a covalent bonding. Thus, it is a qualitative labelling of the silicone oil ensuring that the label is not leaching into the solution independently from the silicone oil.

More preferably, the silicone oil is labelled with at least one BODIPY dye. BODIPY dyes are organoboron compounds which are fluorescent. They strongly absorb light and reemit it in very narrow frequency spreads, with high quantum yields, mostly at wavelengths below 600 nm. They are relatively insensitive to the polarity and pH of their environment and are reasonably stable to physiological conditions. Moreover, they are chemically inert. Thus, they are used as fluorescent dyes, in particular in pharmaceutics.

Most preferably, the silicone oil is labelled with a BODIPY dye by a covalent bonding.

In preferred embodiments of the invention, the fluorescent compound is a BODIPY dye comprising a succinimidyl ester (hereafter abbreviated "NHS ester") group and the silicone oil comprises an amino terminated silicone oil so that the silicone oil and the BODIPY dye are covalently-bound thanks to an amine conjugation.

Indeed, the inventors found that labelling of silicone oil by using an amino terminated silicone oil and a BODIPY dye comprising a NHS ester group was an effective and qualitative method for labelling silicone oil.

An example of an amino terminated silicone oil may be the aminopropyl-terminated PDMS silicone oil, 900-1100 mm²/s marketed by the firm Gelest Inc under the product code DMS-A31, CAS n°106214-84-0. Its density is 0.98 g/cm³ at 25°C and its refractive index is 1.4.

An example of a BODIPY dye comprising an NHS ester group is the BODIPY dye marketed by the firm Invitrogen under the tradename of BODIPY TMR-X NHS Ester and under the product code D6117. The excitation and emission wavelengths of this BODIPY dye are, respectively, 544 nm and 570 nm.

The inventors found a method for labelling the silicone oil which is here below described.

Thus, in these preferred embodiments of the invention, the labelled silicone oil may have been obtained as follows:
a) a BODIPY dye comprising a NHS ester group is mixed in excess with an amino terminated silicone oil in a 1^{st} solvent at ambient temperature, preferably under rotatative agitation, to obtain a 1^{st} mixture containing the labelled silicone oil,
b) a first wash with a 2^{nd} solvent is carried out in order to remove the BODIPY dye in excess (i.e. the BODIPY dye that has not reacted and therefore is not covalently bound to the silicone oil) in the 1^{st} mixture obtained at the end of step a) and to obtain a 2^{nd} mixture,
c) the 1^{st} solvent is evaporated from the 2^{nd} mixture to obtain a 3^{rd} mixture,
d) optionally, a 2^{nd} wash with the 2^{nd} solvent is carried out in order to remove any residual traces of the BODIPY dye in excess,
e) at least one wash with water is further carried out to remove the 2^{nd} solvent,
f) the water is evaporated to retrieve the labelled silicone oil.

The BODIPY dye comprising a NHS ester group and the amino terminated silicone oil have been here above described.

Thus, during the step a), the amino terminated silicone oil and the BODIPY dye covalently bind thanks to an amine conjugation so that a 1^{st} mixture containing the labelled silicone oil is obtained.

The BODIPY dye may be diluted in dimethyl sulfoxide.

The step a) may be carried out during a time comprised between 1 hour and 5 hours.

In step a) of the method of labelling the silicone oil, the 1^{st} solvent may be miscible with the silicone oil and with the BODIPY dye. The 1^{st} solvent may be chosen from the group consisting of tert-butanol, isopropanol, ethanol, acetone, methyl isobutyl ketone, heptane, xylene, hexane, toluene and tertbutyl acetate.

The 2^{nd} solvent is an appropriate solvent for removing the BODIPY dye in excess. The choice of the 2^{nd} solvent may be carried out without any difficulty by a person skilled in the art. Preferably, the 2^{nd} solvent is soluble with the BODIPY dye and not soluble with the labelled silicone oil. The 2^{nd} solvent may be chosen from the group consisting of ethanolamine or any other amine-containing solvent.

Optionally, the 2^{nd} solvent may futher contain water (preferably deionized water). For example, the 2^{nd} solvent may be a mixture of ethanolamine and water (preferably deionized water) or a mixture of any other amine-containing solvent and water (preferably deionized water).

The step c) may be carried out under vacuum at a temperature comprised between 15°C and 50°C during a time comprised 30 minutes and 3 hours.

In the embodiments of the invention in which the 2^{nd} solvent comprises water (preferably deionized water), the aqueous phase may be removed from the 3^{rd} mixture obtained at the end of step c). For example, the aqueous phase may be removed by pipetting.

In some embodiments of the invention, in the mixture of step 1) of the method of evaluating, the silicone oil may be a mixture of silicone oils, at least one of them being a labelled silicone oil, preferably a silicone oil labelled with fluorescent compounds, and more preferably a labelled silicone oil as described above.

Preferably, the silicone oils have similar properties, such as for example the viscosity, the density and/or optical properties (for example the refractive index) in order to have a homogenized mixture of the silicone oils.

For example, the silicone oil is a mixture of the silicone oil marketed by the firm Dow under the tradename of Dow Corning^{®} DC360 and the aminopropyl-terminated PDMS silicone oil, 900-1100 mm²/s marketed by the firm Gelest Inc under the product code DMS-A31, CAS n°106214-84-0.

In these embodiments in which the silicone oil is a mixture of silicone oils, at least one of them being a labelled silicone oil, the silicone oil may comprise in mass % based on the total weight of the silicone oil:
- between 0.5% and 5%, preferably between 0.5% and 2 %, of the labelled silicone oil,
- between 99.5 % and 95%, preferably between 99.5 % and 98%, of the not labelled silicone oil.

In step 1) of the method of evaluating according to the invention, the solvent is an evaporable solvent which is compatible with the substrate. It means that the solvent is chemically inert with the substrate. In other words, the solvent does not degrade the substrate.

Furthermore, the silicone oil is miscible with the solvent.

In step 1) of the method of evaluating according to the invention, the solvent may be chosen from the group consisting of tert-butanol, isopropanol, ethanol, acetone, methyl isobutyl ketone, heptane, xylene, hexane, toluene and tertbutyl acetate.

In the mixture obtained at the end of the step 1), the concentration of the silicone oil may be comprised between 0.0001 g/L and 20 g/L.

In step 2) of the method of evaluating according to the invention, the volume of the mixture in contact with the substrate may be comprised between 10 µL and 200 µL, preferably between 50 µL and 100 µL.

In step 3) of the method of evaluating according to the invention, the evaporation of the solvent may be performed according to any appropriate method and thus may be carried out by a person skilled in the art without any difficulty.

For example, the evaporation of the solvent may be performed in an oven during a time comprised between 30 minutes and 2 hours, preferably between 1 hour and 1 hour 30 minutes, at an appropriate temperature at which the solvent evaporates.

For example, if the solvent is tert-butanol, the step 3) may be performed in an oven for 1 hour at 50°C.

At the end of step 3), the mass of the layer of silicone oil deposited on the substrate may be comprised 5 ng and 1000 µg.

In step 4) of the method of evaluating according to the invention, the volume of the solution in contact with the layer of silicone oil may be comprised 50 µL and 300 µL, preferably between 50 µL and 200 µL.

Preferably, the step 4) is carried out under agitation of the solution, preferably at a speed comprised between 500 rpm and 1200 rpm, more preferably between 900 rpm and 1200 rpm. In this manner, the solution is perfectly homogenized.

Preferably, in step 4), during the contact of the layer of the silicone oil with the solution, said solution is incubated at a temperature comprised between 0°C and 45°C, preferably between 5°C and 40°C.

If in step 5), the adsorption of the compound of interest on the layer of the silicone oil is evaluated, this evaluation may be performed by any appropriate method which is perfectly well-known for a skilled person in the art.

For example (and wihthout being limited to), the evalution of the adsorption of the compound of interest on the layer of the silicone oil may be performed by conducting an assay selected from the group consisting of ELISA, BCA and FRET.

"ELISA" is the abbreviation for Enzyme-Linked ImmunoSorbent Assay.

"BCA" is the abbreviation for BiCinchoninic acid Assay.

"FRET" is the abbreviation for Fluorescence Resonance Energy Transfer.

If in step 5), the degradation of the compound of interest is evaluated, this evaluation may be performed by any appropriate method which is perfectly well-known for a skilled person in the art.

For example (and wihthout being limited to), the degradation of the compound of interest may be performed by conducting an assay selected from the group consisting of chromatography assays, DLS and light obscuration techniques.

"DLS" is the abbreviation for Dynamic Light Scattering.

In preferred embodiments of the invention, the silicone oil is labelled and in step 5) the degradation of the layer of silicone oil is evaluated as follows:
- a determined amount of the solution which was in contact with said layer of the labelled silicone oil is collected and hereafter is called "the collected solution",
- an appropriate amount of a solvent which is miscible both with the labelled silicone oil and the solution is added to the collected solution to obtain a mixture,
- the fluorescence of the mixture is measured,
- the mass of the labelled silicone oil released in the solution is determined thanks to an established calibration curve correlating the measured fluorescence in the collected solution to a mass of the labelled silicone oil released in the collected solution and by taking into account the rate of dilution in collecting of the solution.

The solvent is a solvent which is miscible both with the labelled silicone oil and the solution. Indeed, it guarantees a direct homogenuous and reproducible fluorescence readout.

This solvent may be chosen amongst the solvents that have been described for the step 1) of the method of evaluating according to the invention. Preferably, the solvent is tert-butanol.

This step of mixing the collected solution with such a solvent is necessary for homogenizing said collected solution prior performing the measurement of fluorescence. Indeed, when silicone oil (in particular labelled silicone oil) is present in aqueous phase (such as the collected solution which may be a pharmaceutical solution), it forms round droplets of different sizes due to the non-miscibility of the oil in water. Direct fluorescence measurements of silicone oil droplets in water would produce highly variable results as the fluorescent silicone oil droplets being free to move in the collected solution, would create very inhomogeneous samples for measurements of fluorescence.

The calibration curve may be established as follows: the fluorescence of different solutions of a mixture of water and a solvent which is miscible both with the labelled silicone oil and water, said solutions further containing known masses (from 0 to any appropriate amount, for example 1000 µg) of the labelled silicone oil, is measured.

For example, (and wihthout being limited to), the solution may be 50% in mass tert-butanol and 50% in mass water.

The fluorescence background value, corresponding to a solution without labelled silicone oil, is subtracted to all the measured fluorescence values priorly the conversion in mass.

Then, thanks to the measurement of fluorescence of the collected solution and the calibration curve, the amount of the released labelled silicone oil present in the collected solution is determined.

Then, by taking into account the rate of dilution in collecting of the solution, it is possible to evaluate the mass of the released labelled silicone oil in the solution from this determined amount of the released silicone oil present in the collected solution.

For example, if the volume of the solution is 200 µL and the volume of the collected solution is 100 µL. The rate of dilution in collecting of the solution is ½. Thus, the amount of the released labelled silicone oil in the solution is twice the determined mass of released labelled silicone oil in the collected solution.

The mass of the labelled silicone oil released in the solution constitutes an evaluation of the degradation of the layer of the silicone oil.

As explained above, the substrate may be any device or any part of a device on which a layer of silicone oil may be deposited. For example, the substrate may be chosen from the group consisting of syringes, pump pipes, well plates (in particular glass bottom well plates).

The method of evaluating according to the present invention may be very useful if the substrate is a syringe. In particular, the evaluation of the degradation of the layer of silicone oil deposited on the barrel and on the stopper surfaces with a labelled silicone oil as described above is original and convenient to be carried out.

Moreover, as explained above, the experiments for evaluating the interactions between a given layer of silicone oil and a given pharmaceutical solution which are directly performed in the syringes may be expensive, long and complicated.

Thus, in order to avoid these costsly and long experiments, it may be helpful, in the context of preliminary experiments, to carry out the method of evaluating according to the present invention with glass bottom well plates as the substrate for evaluating said interactions. Indeed, these preliminary experiments performed with glass bottom well plates will micmic the interactions between the layer of silicone oil and the pharmaceutical solution that will occur in the syringe. Thus, these preliminary experiments performed according to the method of evaluating of the present invention can provide guidance by giving some results of the predicted behavior of the tested pharmaceutical solution in the syringe.

Thus, it explains why in most preferred embodiments of the embodiments of the invention, the substrate is glass bottom well plates. This substrate provides the following advantages to the method of evaluating according to the invention:
- the method is reliable, unexpensive and is easily carried out;
- the method allows the easy creation of a lot of replicates;
- the method is compatible with standard lab equipment (such as agitators, incubators and fluorescence plate readers).

The present invention is illustrated by the following example.

### EXAMPLE

### Labelling of the aminopropyl-terminated PDMS silicone oil

The labelling of silicone oil was carried out as follows:
Step a): 80 µL of 10 mmol/L of the above mentioned BODIPY TMR-X NHS Ester dye diluted in dimethyl sulfoxide was mixed with 1 mL of the above mentioned aminopropyl-terminated PDMS silicone oil, 900-1100 mm²/s marketed by the firm Gelest Inc in 4 mL of tert-butanol (i.e. the 1^{st} solvent) for 3 hours at ambient temperature under rotative agitation to obtain a 1^{st} mixture.
Step b): A first wash with a solution of 150 mmol/L of ethanolamine in deionized water (i.e. the 2^{nd} solvent) was carried out in order remove the BODIPY dye in excess in the 1^{st} mixture obtained at the end of step a) to obtain a 2^{nd} mixture. The BODIPY dye in excess is the one that has not covalently reacted with the amino terminated silicone oil.
Step c): The 2^{nd} mixture was transferred into Eppendorf tubes^{®} and the tert-butanol was evaporated by vacuum during 1 hour and 30 minutes at a temperature of 37°C by using a device marketed by the firm Eppendorf under the tradename of Vacufuge plus Vacuum concentrator in order to obtain a 3^{rd} mixture.
   Given that the 2^{nd} solvent comprised deionized water, the aqueous phase was removed from the 3^{rd} mixture obtained at the end step c) by pipetting.
Step d): A 2^{nd} wash with the solution of 150 mmol/L of ethanolamine in deionized water was performed in order to remove any residual traces of the BODIPY dye in excess.
Step e): 3 water washing steps were performed to remove the potential ethanolamine traces.
Step f): The water has been evaporated in order to obtain the labelled silicone oil.

The silicone oil was labelled with a labelling rate of 0.03%, estimated thanks to the concentration of the BODIPY dye measured by absorbance, and considering the molar ratio of 2:1, respectively for the BODIPY dye and for the aminopropyl-terminated PDMS silicone oil.

This labelling rate is perfectably convenient for fluorescence experiments in order to determine in a reliable manner the amount of silicone oil present in a sample of a pharmaceutical solution.

Controls of the fluorescence of the BODIPY dye were performed before and after the labelling of silicone oil. The controls confirmed the preservation of the fluorescence properties of the BODIPY dye, once grafted to the silicone oil.

### Preparation of the mixture of silicone oils which comprises a not labelled PDMS silicone oil and the thus obtained labelled silicone oil

The obtained labelled silicone oil was mixed at 1% in mass with the above mentioned Dow Corning^{®} DC360 silicone oil during 3 hours at ambient temperature under rotative agitation to obtain the mixture of silicone oils, hereafter called "the labelled silicone oil".

It means that this obtained labelled silicone oil comprised, in mass % based on its total weight: 99% of the not labelled silicone oil and 1% of the labelled silicone oil.

More precisely, the labelled silicone oil was used for carrying out the method of the present invention for evaluating the interactions between a layer of this labelled silicone oil deposited on bottom of glass-bottom 96-well plates with different solutions to be tested which are here below detailed.

### Description of the tested solutions:

The tested solutions were:
A) solutions containing different nonionic surfactants at differents concentrations in order to evaluate the release of silicone oil in such solutions,
B) solutions containing these different nonionic surfactants and a drug which was trastuzumab, i.e. a humanized immunoglobulin G isotype 1 (abbreviated "IgG1") provided by the firm Promise Proteomics at a concentration of 5 g/L with an average molecular mass of 145 529 Da in order to evaluate the release of silicone oil in such solutions.

The nonionic surfactants were:
- polysorbate 80 (hereafter abbreviated "PS80"),
- polysorbate 20 (hereafter abbreviated "PS20"),
- poloxamer 188 (hereafter abbreviated "P188").

A 10 mmol/L of L-histidine (marketed by the firm Merck under the tradename Sigma, Product code H3911) at pH 6 containing 2 % in mass of trehalose (marketed by the firm Roth under the Reference number 5151) was prepared and is abbrievated here after as "the buffer".
A) The solutions containing the different nonionic surfactants were as follows:
   - Solution A0 was a solution of the buffer,
   - Solution A1 was a solution of the buffer which further contained PS20 at a concentration of 100 ppm,
   - Solution A2 was a solution of the buffer which further contained PS20 at a concentration of 200 ppm,
   - Solution A3 was a solution of the buffer which further contained PS20 at a concentration of 400 ppm,
   - Solution A4 was a solution of the buffer which further contained PS80 at a concentration of 100 ppm,
   - Solution A5 was a solution of the buffer which further contained PS80 at a concentration of 200 ppm,
   - Solution A6 was a solution of the buffer which further contained PS80 at a concentration of 400 ppm,
   - Solution A7 was a solution of the buffer which further contained P188 at a concentration of 400 ppm,
   - Solution A8 was a solution of the buffer which further contained P188 at a concentration of 800 ppm,
   - Solution A9 was a solution of the buffer which further contained P188 at a concentration of 10 000 ppm.
B) The solutions containing the different nonionic surfactants and the drug were as follows:
   - Solution B0 was a solution of the buffer which further contained the drug at a concentration of 1 g/L,
   - Solution B1 was a solution of the buffer which further contained the drug at a concentration of 1 g/L and PS20 at a concentration of 200 ppm,
   - Solution B2 was a solution of the buffer which further contained the drug at a concentration of 1 g/L and PS80 at a concentration of 200 ppm,
   - Solution B3 was a solution of the buffer which contained the drug at a concentration of 1 g/L P188 at a concentration of 800 ppm.

### Step 1 of the method : Preparation of the mixture of the labelled silicone oil with tert-butanol

The labelled silicone oil was mixed with tert-butanol at a concentration of 20 g/L to obtain a mixture.

### Step 2 of the method : Contact of the mixture with the substrate

The substrate was glass-bottom 96-well plates marketed by the firm Cellvis under the tradename P96-0-N.

50 µL of the mixture were put in the glass-bottom 96-well plates.

### Step 3 of the method: Evaporation of the solvent

The glass-bottom 96-well plates containing the mixture were put in an oven for 1 hour at 50°C until complete evaporation of the tert-butanol to obtain samples coated with a layer of the silicone oil having a mass of 1 mg.

### Step 4 of the method: Contact of the layer of the labelled silicone oil with the tested solutions

For each tested solution, 200 µL of the tested solution were added into a sample coated with a layer of the labelled silicone oil having a mass of 1 mg. 12 replicates were prepared for each tested solution.

All the samples coated with a layer of the labelled silicone oil and containing a tested solution were incubated at 37°C and the tested solutions were maintained under agitation at 1200 rpm until the below detailed analysis.

### Step 5) of the method: Evaluation of the release of the labelled silicone oil

The samples were analyzed at indicated times (0, 4 hours and 8 hours after the beginning of the incubation) in order to evaluate the mass of the labelled silicone oil which was released in the tested solution at said indicated times.

At each time point, 100 µL of the tested solution (i.e. the "collected tested solution") were collected from the sample and put in a black polystyrene well.

100 µL of tert-butanol were added into the black polystyrene well in order to dissolve the labelled silicone oil and thus to increase the homogenization of the sample to be analyzed prior to reading the fluorescence (excitation and emission wavelengths of the BODIPY dye were, respectively, 544 nm and 570 nm and the bandwidth was 10 nm). Tert-butanol is miscible with both the labelled silicone oil and the buffer which is water-based.

The fluorescence was measured with a spectrophotometer (plate reader).

In order to determine the mass of the labelled silicone oil released in the collected tested solution on the basis of the measured fluorescence, a calibration curve correlating the measured fluorescence in the collected tested solution to a mass of the labelled silicone oil released in the collected tested solution had been established.

The calibration curve was established as follows: the fluorescence of different solutions (50% in mass tert-butanol and 50% in mass of water) which contained known masses (0 to 1000 µg) of the labelled silicone oil was measured. The fluorescence, corresponding to a solution without labelled silicone oil, was measured at 11.92 +/- 0.79 AU. This value was subtracted to all the measured fluorescence values prior to the conversion in mass.

Thus, thanks to the measurement of fluorescence in the collected tested solution and the calibration curve, the amount of released labelled silicone oil present in the 100 µL collected tested solution was determined.

Then, by applying the hypothesis that the amount of the released labelled silicone oil in the tested solution is twice the amount of said determined released silicone oil present in the 100 µL collected tested solution, it is possible to calculate said amount of the released labelled silicone oil in the tested solution.

The table 1 details, at times 0, 4 hours and 8 hours, the average mass (µg) of the labelled silicone oil released in the tested solutions A0 to A9 and the standard deviation.

**Table 1**

| **Tested solution** | **0** | | **4 hours** | | **8 hours** | |
|---|---|---|---|---|---|---|
| | **Average (µg)** | **Standard deviation** | **Average (µg)** | **Standard deviation** | **Average (µg)** | **Standard deviation** |
| A0 | 0 | 0 | 0.16 | 0.57 | 0.16 | 0.57 |
| A1 | 0 | 0 | 2.56 | 2.01 | 3.24 | 2.55 |
| A2 | 0.02 | 0.06 | 9.09 | 1.09 | 7.64 | 2.22 |
| A3 | 0.02 | 0.06 | 10.73 | 2.70 | 10.26 | 2.22 |
| A4 | 0.00 | 0.00 | 6.06 | 2.22 | 6.76 | 2.76 |
| A5 | 0.02 | 0.06 | 7.69 | 1.38 | 6.53 | 3.19 |
| A6 | 0.00 | 0.00 | 12.13 | 5.62 | 10.03 | 4.05 |
| A7 | 1.71 | 3.12 | 0.00 | 0.00 | 0.06 | 0.10 |
| A8 | 1.28 | 2.58 | 0.00 | 0.00 | 0.62 | 1.13 |
| A9 | 0.56 | 1.66 | 0.27 | 0.87 | 2.75 | 4.41 |

The results detailed in the table 1 show:
- The tested solution A0 which contained only the buffer indicates that the labelled silicone oil release is null overtime,
- Regarding the tested solutions A1 to A3 which further contained PS20 at different concentrations: at initial time, the release of the labelled silicone oil is null whatever the concentration of the PS20. Then, at 4 hours, the mass of the released labelled silicone oil increases significantly. The mass increase is not proportional to the PS20's concentration and does not evolve significantly between 4 hours and 8 hours.
- Regarding the tested solutions A4 to A6 which further contained PS80 at different concentrations: the initial values show that no labelled silicone oil is released whatever the concentration of PS80. Then, similarly to the tested solutions containing PS20, the mass increase is not proportional to the concentration nor the time.
- Regarding the tested solutions A7 to A9 which further contained P188: there is no significant release of the labelled silicone oil depending on the concentration and the time.

The table 2 details, at times 0, 4 hours and 8 hours, the average mass (µg) of the labelled silicone oil released in the tested solutions B0 to B3 and the standard deviation.

**Table 2**

| **Tested solution** | **0** | | **4 hours** | | **8 hours** | |
|---|---|---|---|---|---|---|
| | **Average (µg)** | **Standard deviation** | **Average (µg)** | **Standard deviation** | **Average (µg)** | **Standard deviation** |
| B0 | 2.59 | 3.66 | 1.15 | 3.13 | 2.61 | 5.48 |
| B1 | 2.96 | 4.76 | 1.51 | 1.79 | 3.94 | 3.80 |
| B2 | 2.97 | 6.82 | 3.50 | 2.78 | 4.38 | 4.17 |
| B3 | 3.85 | 9.19 | 0.00 | 0.00 | 0.04 | 0.08 |

The results detailed in the table 2 show:
- Regarding the tested solution B0 and in comparison with the tested solution A0, the presence of the drug slightly increases the release of the labelled silicone oil, but it does not evolve with time,
- Regarding the tested solutions B1 and B2 (i.e. the tested solutions which respectively contained PS20 and PS80), the presence of the drug reduces the release of the labelled silicone oil,
- Regarding the tested solution B3 (i.e. the tested solution which further contained P188), the presence of the drug does not have an impact on the release of the labelled silicone oil.

Thus, these experiments performed according to the method of evaluating of the present invention and by using glass-bottom 96-well plates as the substrate provide some very interesting guidance by giving some results of the predicted behavior of the above detailed tested solutions in a syringe in which the barrel and stopper surfaces are coated with silicone oil.

## Claims

1. A method of evaluating the interactions between a layer of silicone oil deposited on a substrate and at least one compound of interest (preferably a drug), which is contained in a solution (preferably a pharmaceutical solution), said solution being in contact with the silicone oil, which comprises at least the following steps:
1) preparing a mixture containing at least the silicone oil and an evaporable solvent which is miscible with the silicone oil and compatible with the substrate,
2) contacting the mixture with the substrate,
3) evaporating the solvent to obtain a substrate coated with a layer of the silicone oil,
4) contacting the layer of the silicone oil with the solution containing the at least one compound of interest,
5) evaluating at least one of the following parameters i), ii) and iii) :
i) the adsorption of the at least one compound of interest on the layer of the silicone oil,
ii) the degradation of the at least one compound of the interest,
iii) the release of the silicone oil in the solution,
wherein if the parameter iii) of the release of the silicone oil in the solution is evaluated, the silicone oil is labelled.

2. The method of evaluating according to claim 1, **characterized in that** the solution is an aqueous pharmaceutical solution.

3. The method of evaluating according to claim 1 or claim 2, **characterized in that** the compound of interest is a drug which is selected from the groupe consisting of therapeutic proteins (such as monoclonal antibodies), vaccines, drug-conjugates, oligonucleotides, drug carriers (such as lipid nanoparticles) and oncolytic virus.

4. The method of evaluating according to any one of claims 1 to 3, **characterized in that** the material of the substrate is chosen from the group consisting of glass, rubber, plastics and metals.

5. The method of evaluating according to any one of claims 1 to 4, **characterized in that** the substrate is chosen from the group consisting of syringes, pump pipes, well plates, glass or plastics or rubber slices/plates, plunger rubber stoppers and needles.

6. The method of evaluating according to claim 5, **characterized in that** the substrate is a glass bottom well plate or a syringe.

7. The method of evaluating according to any one of claims 1 to 6, **characterized in that** in step 1) the solvent is chosen from the group consisting of tert-butanol, isopropanol, ethanol, acetone, methyl isobutyl ketone, heptane, xylene, hexane, toluene and tertbutyl acetate.

8. The method of evaluating according to any one of claims 1 to 7, **characterized in that** in the mixture obtained at the end of the step 1), the concentration of the silicone oil is comprised between 0.0001 g/L and 20 g/L.

9. The method of evaluating according to any one of claims 1 to 8, **characterized in that** the silicone oil has a viscosity comprised between 50 mm²/s and 1 000 000 mm²/s at 25°C.

10. The method of evaluating according to any one of claims 1 to 9, **characterized in that** the silicone oil comprises a medical grade polydimethylsiloxane silicone oil.

11. The method of evaluating according to any one of claims 1 to 10, **characterized in that** the silicone oil is labelled with a fluorescent compound by a covalent bonding.

12. The method of evaluating according to claim 11, **characterized in that** said fluorescent compound is a BODIPY dye comprising a succinimidyl ester group and **in that** said silicone oil comprises an amino terminated silicone oil so that the silicone oil and the BODIPY dye are covalently-bound thanks to an amine conjugation.

13. The method of evaluating according to claim 12, **characterized in that** the labelled silicone oil has been obtained as follows:
a) a BODIPY dye comprising a succinimidyl ester group is mixed in excess with an amino terminated silicone oil in a 1^{st} solvent at ambient temperature, preferably under rotatative agitation, to obtain a 1^{st} mixture containing the labelled silicone oil,
b) a first wash with a 2^{nd} solvent is carried out in order to remove the BODIPY dye in excess in the 1^{st} mixture obtained at the end of step a) and to obtain a 2^{nd} mixture,
c) the 1^{st} solvent is evaporated from the 2^{nd} mixture to obtain a 3^{rd} mixture,
d) optionally, a 2^{nd} wash with the 2^{nd} solvent is carried out in order to remove any residual traces of the BODIPY dye in excess,
e) at least one wash with water is further carried out to remove the 2^{nd} solvent,
f) the water is evaporated to retrieve the labelled silicone oil.

14. The method of evaluating according to any one of claims 1 to 13, **characterized in that** the silicone oil is a mixture of silicone oils, at least one of them being a labelled silicone oil and said silicone oil comprises in mass % based on the total weight of the silicone oil :
- between 0.5% and 5%, preferably between 0.5% and 2 %, of the labelled silicone oil,
- between 99.5 % and 95%, preferably between 99.5 % and 98%, of the not labelled silicone oil.

15. The method of evaluating according to any one of claims 1 to 14, **characterized in that** the silicone oil is labelled and in step 5) the degradation of the layer of silicone oil is evaluated as follows:
- a determined amount of the solution which was in contact with said layer of the labelled silicone oil is collected and hereafter is called "the collected solution",
- an appropriate amount of a solvent which is miscible both with the labelled silicone oil and the solution is added to the collected solution to obtain a mixture,
- the fluorescence of the mixture is measured,
- the mass of the labelled silicone oil released in the solution is determined thanks an established calibration curve correlating the measured fluorescence in the collected solution to a mass of the labelled silicone oil released in the collected solution and by taking into account the rate of dilution in collecting of the solution.
